# EUROPEAN PATENT APPLICATION

(11) **EP 1 249 206 A1**
(43) Date of publication of application: **16.10.2002**
(21) Application number: 01109225.1
(22) Date of filing: 14.04.2001
(51) Int. Cl.: A61B 5/117, G09F 3/00

(54) **Measurement of patient data requiring operator and patient identification**

(71) Applicant: Agilent Technologies, Inc. (a Delaware corporation), Palo Alto, CA 94303 (US)
(72) Inventor: Boos, Andreas, 71149 Bondorf (DE)
(74) Representative: Barth, Daniel

(57) **Abstract**

Disclosed is a system for measuring a physiological parameter of a patient. A measuring means (12) measures a signal representative for the physiological parameter of the patient, and an operator identification means (10) provides an operator identification signal for identifying an operator performing or helping to perform the measurement. The measured signal is associated with the operator identification signal for identifying the measured signal and thus providing an operator identified measuring signal (14).

## Description

### BACKGROUND OF THE INVENTION

The present invention relates to the measurement of a physiological parameter of a patient.

Usually measurement of a physiological parameter of a patient is performed in a hospital or in a nursing home. It is also known to transfer care services from hospital to out-of-hospital settings, e.g. at home or in remote locations using Telemedicine solutions. Telemedicine is defined as "medicine at a distance", thus enabling care providers to diagnose and treat patients at their point of need. Technically the Telemedicine solution can consist of monitoring devices deployed in the patients home. These measure vital signs, and transmit the recorded data via existing media (e.g. the phone line) to a care provider.

In a lot of cases the patient can perform these measurements alone, without help of a third person. But there also exist a lot of cases, in which an operator is necessary, e.g. in an intensive care unit or with respect to complex measurements. In this context an operator is e.g. a doctor, physician, assistant, nurse or male nurse.

In the field of care services exists always the requirement to improve the reliability of performed measurements and also the possibility to check the measurements. Therefore it is useful to have a security and/or quality management system, which documents the operator performing or helping to perform the measurement. To reach this aim it is important to unambiguously assign a recorded measurement signal to a specific operator.

From the field of Telemedicine a solution is known to provide a correlation of the measured patient vital signs with previously recorded data for assigning recorded measurement signals to a specific individual. This concept takes advantage of the fact that normally physiological parameters only change within specific limits. Data recorded exhibiting more dramatic fluctuations than physiologically possible can thus clearly be detected and eliminated, because they cannot stem from the patient. This concept, however, does not support the recognition of erroneous vital sign measurements that are within the physiological range of the patient, but not generated by that patient. The solution is dependent on the availability of (previous) baseline data, and also does not allow numerous patients to intentionally use the same devices.

From PCT/EP99/10346 of 23.12.1999 is known a method for measuring a physiological parameter of a patient. This method comprises the steps of measuring a signal representative for the physiological parameter of the patient, providing an patient identification signal for identifying the patient, and combining and/or associating the measured signal with the patient identification signal for identifying the measured signal. This patient identification signal provides an unambiguous patient identifier, allowing remote recognition of the patient performing the measurement.

### SUMMARY OF THE INVENTION

It is an object of the invention to unambiguously assign a recorded measurement signal to a specific operator. The object is solved by the independent claims. Preferred embodiments are shown by the dependent claims.

According to the invention, a measurement signal of a patient is provided with an operator identification signal for clearly identifying the specific person performing or helping to perform the measurement. The operator identification signal provides an unambiguous operator identifier, allowing remote recognition of the operator performing or helping to perform the measurement.

According to a preferred embodiment of the invention, the operator identified measurement signal of a specific patient is additionally provided with a patient identification signal for clearly identifying the specific patient to which the measurement is referring to.

The operator and/or patient identification signal can preferably be provided e.g. by:
- a user-initiated entry of a specific operator and/or patient identifier (e.g. key-code, smart cards),
- an automatic sensing e.g. of tags worn by the operator and/or patient,
- an evaluation of characteristic biological features (e.g. fingerprint, voice) of the operator and/or patient.

For combining/associating (or linking) the operator and/or patient identification signal with the measurement signal, the measurement equipment comprises an operator identification reader (OIR) and/or a patient identification reader (PIR). In a close temporal relationship to the actual physiological signal measuring process, e.g. during the measuring process or (shortly) before or after the measuring process, however, at least before a succeeding measuring process, the operator and/or patient will be identified, and the OIR and/or PIR can provide the operator identification and/or patient identification to the measurement equipment, which again can associate the operator and/or patient identification signal with the measurement signal. The operator and/or patient can be identified by sending the identification signal, or any kind of operator and/or patient identifier from which the operator and/or patient identification signal can be derived from, to the OIR and/or PIR.

The OIR and/or PIR might probe whether the person performing the current measurement has a valid identification. Preferably only when the OIR and/or PIR recognizes a valid identifier, it will (electronically) attach the operator and/or patient identification signal to the patient's measured parameter(s). Thus, each patient signal measured can be explicitly assigned to a specific operator and/or patient. Alternatively, if the person performing the measurement has no valid identifier, the measurement can be discarded.

In a first aspect of the invention, the operator and/or patient identification signal will be associated with the measurement signal by an active (preferably user initiated) interaction of the operator and/or the patient. In a preferred embodiment, operators and/or patients are required to enter the operator and/or patient identification signal or an operator and/or patient identifier from which the operator and/or patient identification signal can be derived, such as a key code or a smart card, e.g. either before or after conducting a measurement. This requires that the respective measurement device is equipped with some form of key-code or a smart card reader interface. Additionally, it demands significant operator and/or patient interaction, by having to remember the correct key-code, as well as the sequence in which all these activities (physiologic measurement and key-code entry) have to be performed. Potentially, this mechanism of identifying the operator and/or patient excludes specific groups of users, such as people with mental disabilities.

In a second aspect of the invention, the operator and/or patient identification signal will be automatically associated with the measurement signal. The measurement equipment comprises the operator and/or patient identification reader OIR and/or PIR, and the operator and/or patient carries or is equipped (e.g. in form of a bracelet or clip) with a corresponding operator and/or patient identification device (OID and/or PID). The OIR and/or PIR and the OID and/or PID are adapted to establish a communication therebetween, at least in a close temporal relationship to the actual physiological signal measuring process.

During the communication, the OID and/or PID identifies the operator and/or patient, and the OIR and/or PIR can provide the operator and/or patient identification to the measurement equipment, which again can associate the operator and/or patient identification signal with the measurement signal. The OID and/or PID can identify the operator and/or patient by sending the operator and/or patient identification signal, or any kind of operator and/or patient identifier from which the operator and/or patient identification signal can be derived from, to the OIR and/or PIR. In a preferred embodiment, the OIR and/or PIR sends out an electrical signal or scans (e.g. barcode, fingerprint etc.) the OID and/or PID in chronological context with the physiological measurement.

The communication between the operator and/or patient identification device OID and/or PID and the operator and/or patient identification reader OIR and/or PIR can be provided by any means known in the art, such as wired or wireless transmission (e.g. using ultrasound, infrared, magnetic, radio frequency, optical, or other electro-magnetic transmission types).

The OIR and/or PIR might then probe whether the person performing the current measurement has a valid identification. When the OIR and/or PIR recognizes an eligible OID and/or PID, it registers its operator and/or patient identifier (e.g. a unique identification number), and electronically attaches the operator and/or patient identification signal (e.g. this unambiguous identification number) to the patient's measured parameter(s).

In a preferred embodiment, the OIR and/or PIR comprises a sensing device capable of reading the operator and/or patient identifier from the OID and/or PID and combining the data recorded by the physiological measurement equipment and the unique operator and/or patient identification to an information package that is clearly assigned to one specific operator and/or patient.

The sensing device can be implemented utilizing a variety of technologies, ranging from optical solutions (e.g. barcode readers, infra-red), wireless electrical concepts (e.g. RF transponders, magnetic coupling), to bio-sensing devices e.g. capable of analyzing the operator's and/or patient's finger print, voice characteristics or other unique biological markers. In the latter case the operators and/or patients personal characteristics (e.g. fingerprint, voice etc.) are utilized as OIDs and/or PIDs to correlate physiological measurements to specific individuals.

The OIR and/or PIR can be an integral part of the measurement equipment or only connected thereto. The OIR and/or PIR preferably probes if the individual performing or helping to perform a measurement has a valid operator and/or patient identifier of the OID and/or PID, and if recognition is positive, links this recorded identification with the measured signal.

In a preferred embodiment of an Automatic Operator Identification System (AOIS) and/or of an Automatic Patient Identification System (APIS), the OID and/or the PID comprises a compact transponder that carries a tamper-proof operator and/or patient identification number. This transponder can be packaged in a variety of sizes. It is preferably ultra thin and battery free, allowing it to be worn by the operator and/or patient, for example as a bracelet or a clip. When the operator and/or patient performs a measurement, the OIR and/or the PIR of the measuring device automatically interrogates the user's transponder e.g. by a radio frequency (RF) reader. This implies that the transponder does not have to be in direct mechanical contact with the measurement device. The operator and/or the patient, however, needs to be in proximity to the OIR and/or the PIR for the reader to link up with the transponder. If the measurement unit detects a valid transponder it transmits the operator and/or patient identification signal (e.g. an operator and/or a patient identification number) together with the measured vital signs to the care provider. Instead of using an RF link between the transponder and the reader, this concept could also be implemented using e.g. magnetic coupling.

This solution requires no intervention by the operator and/or the patient other than wearing the transponder, and initiation of the measurement. Interrogation of eligible users and transmission of the recorded identification, as well as the patients measured physiological parameters is a totally automatic process performed by the measurement unit. It is clear that the invention can be partly or entirely embodied by one or more suitable software programs, which can be stored on or otherwise provided by any kind of data carrier, and which might be executed in or by any suitable data processing unit.

This concept allows physiological measurements to be clearly assigned to one operator and/or to one patient. It also enables filtering of measurements performed by individuals with valid or no identifier. Additionally, numerous people (operator and/or patient) can use the same set of measurement devices, by electronically tagging each recorded physiological signal, linking it to one specific operator and/or patient.

### BRIEF DESCRIPTION OF THE DRAWINGS

Other objects and many of the attendant advantages of the present invention will be readily appreciated and become better understood by reference to the following detailed description when considering in connection with the accompanied drawings. Features that are substantially or functionally equal or similar will be referred to with the same reference sign(s).

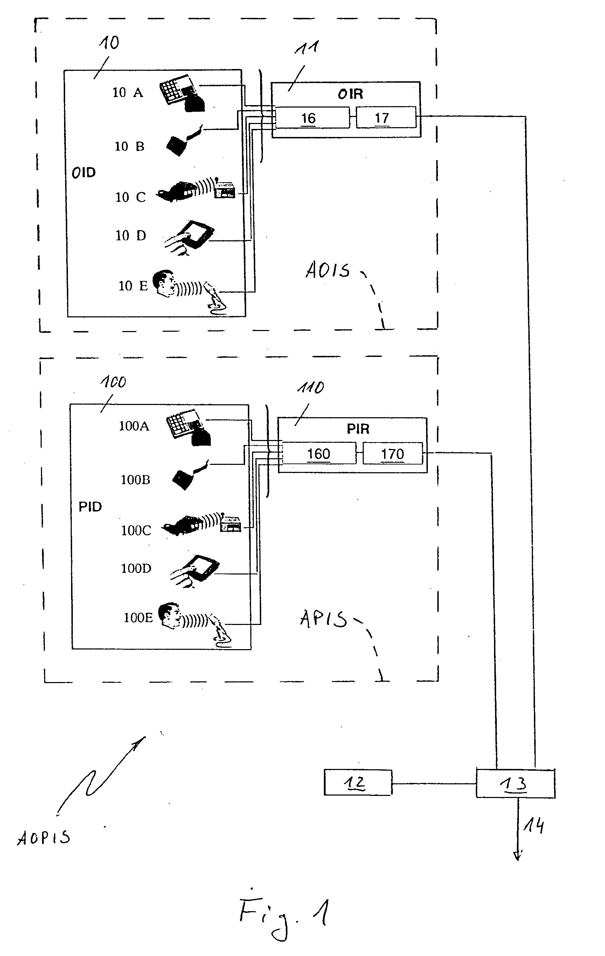

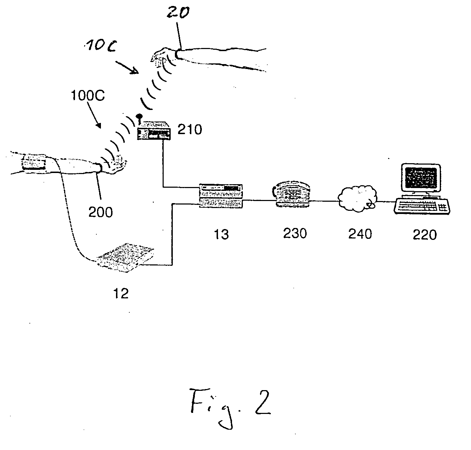

- Figs. 1 and 2: depict an Automatic Operator and Patient Identification System (AOPIS) as an example of an embodiment according to the present invention.

### DETAILED DESCRIPTION OF THE INVENTION

Fig. 1 depicts an Automatic Operator and Patient Identification System (AOPIS) as an example of an inventive embodiment. This AOPIS comprises an Automatic Operator Identification System (AOIS) and an Automatic Patient Identification System (APIS) each symbolized with a frame of broken lines. In the AOIS an Operator Identification Device (OID) 10 is coupled with an Operator Identification Reader (OIR) 11 for identifying a patient's measuring results provided by a physiological measurement unit 12. For clearly identifying the specific operator performing or helping to perform the measurement, the OID 10 provides an unambiguous operator identifier for the operator to the OIR 11. The OIR 11, again, checks the operator identifier from the DID 10 and sends an operator identification signal to a signal packaging unit 13 which further receives the patient's measuring results from the physiological measurement unit 12.

Furthermore in the APIS a Patient Identification Device (PID) 100 is coupled with a Patient Identification Reader (PIR) 110 for additionally identifying the patient's measuring results provided by the physiological measurement unit 12. For clearly identifying the specific patient performing the measurement or for which the measurement is performed, the PID 100 provides an unambiguous patient identifier for the patient to the PIR 110. The PIR 110, again, checks the patient identifier from the PID 100 and sends a patient identification signal to the signal packaging unit 13 which further receives the patient's measuring results from the physiological measurement unit 12 and the operator identification signal from the OIR.

The signal packaging unit 13 combines the patient's measuring results with the operator identification signal and with the patient identification signal to a combined measurement and ID output signal 14.

Fig. 1 shows a plurality of different solution for the OID 10 and the PID 100, which can be applied either individually or in combination. OID 10A and PID 100A represent a keypad entry for manually typing in an operator identifier or a patient identifier, respectively, such as a PIN code. OID 10B and PID 100B represent a barcode solution for reading a barcode or the like containing an operator identifier or a patient identifier, respectively. OID 10C and PID 100C show a transponder concept, wherein an operator identifier and a patient identifier, respectively, is registered via an RF link between a transponder worn or otherwise held by the operator and/or by the patient and a receiving unit (which might also be part of the OIR 11 and/or PIR 110). OIDs 10D and 10E and PIDs 100D and 100E represent identification systems based on identification by physical properties of the operator and/or patient, such as fingerprint (10D and100D) or voice (10 E and 100E).

The OIR 11 preferably comprises a reading unit 16 for reading the operator identifier from the OID 10 and an operator identifier evaluation unit 17 for validating the operator identifier. Likewise the PIR 110 preferably comprises a reading unit 160 for reading the patient identifier from the PID 100 and a patient identifier evaluation unit 170 for validating the patient identifier. Ina preferred embodiment OIR and PIR are arranged in a common housing.

Fig. 2 depicts a more conceptual view of the Automatic Operator and Patient Identification System (AOPIS) as depicted in Fig.1 for the example of the OIP 10C and the PID 100C. The OIP 10C comprises a transponder 20 worn e.g. at the operator's arm. Likewise the PID 100C comprises a transponder 200 worn e.g. at the patient's arm. OID 10C and PID 100C additionally comprise a common transmission unit 210, which is preferably integrated in the OIR 11 or in the PIR 110, for establishing a radio frequency communication therebetween. The transmission unit 210 is equipped with a radio frequency transmitter/receiver and an antenna. Before or during a measurement, the OIR 11 and the PIR 110 send out a signal (via the transmission unit 210) probing if the person performing the measurement is wearing an eligible transponder 20 and 200, respectively. The low energy signal transmitted by the transmission unit 210 activates the transponder 20 and 200, respectively, thus each of the transponders 20 and 200 does not need to be powered by itself (e.g. by a battery). The activation signal from the transmission unit 210 also instructs the transponder 20 and 200, respectively, to transmit back its stored operator identifier and patient identifier, respectively, such as an operator identification number and a patient identification number, respectively.

The reading unit 16 reads out the operator identifier from the transmission unit 210. If the OIR 11 comprises the operator identifier evaluation unit 17, it will validate the operator identifier whether it is eligible. If the operator identifier is eligible, the OIR 11 sends an operator identification signal derived from the operator identifier to the signal packaging unit 13. Likewise the reading unit 160 reads out the patient identifier from the transmission unit 210. If the PIR 110 comprises the patient identifier evaluation unit 170, it will validate the patient identifier whether it is eligible. If the patient identifier is eligible, the PIR 110 sends a patient identification signal derived from the patient identifier to the signal packaging unit 13. The signal packaging unit 13, which further receives the patient's measuring results from the physiological measurement unit 12, combines all three signals and sends the combination to a remote user 220 (e.g. a reviewing clinician), preferably via a modem 230 and a telecommunication system 240. Alternatively, the signal packaging unit 13 may simply package the operator identification signal, the patient identification signal and the measured signal and transmits this information to the remote user 220.

## Claims

1. A method for measuring a physiological parameter of a patient, the method comprising the steps of:
(a) measuring a signal representative for the physiological parameter of the patient,
(b) providing an operator identification signal for identifying an operator performing or helping to perform the measurement, and
(c) combining and/or associating the measured signal with the operator identification signal for identifying the measured signal with respect to the operator.

2. The method of claim 1, comprising the steps of:
(d) providing a patient identification signal for identifying the patient, and
(e) combining and/or associating the measured signal with the patient identification signal for identifying the measured signal with respect to the patient.

3. The method of claim 1 or 2, wherein step (b) and/or (d) is/are executed in a close temporal relationship to step (a), preferably during the measuring process or before or after the measuring process.

4. The method of any one of the above claims, wherein step (b) and/or (d) is/are executed before a succeeding measuring process.

5. The method of any one of the above claims, wherein step (c) and/or (e) comprises/comprise the steps:
(c1) probing whether the operator identification signal is valid, and when the operator identification signal is valid:
(c2) combining and/or associating the measured signal with the operator identification signal;
otherwise, discarding the measurement, and/or
(e1) probing whether the patient identification signal is valid, and
when the patient identification signal is valid:
(e2) combining and/or associating the measured signal with the patient identification signal;
otherwise, discarding the measurement.

6. The method of any one of the above claims, wherein step (b) and/or (d) comprises/comprise a step of:
(b1) providing the operator identification signal by an active interaction of the operator or a third person, preferably by entering the operator identification signal or an operator identifier from which the operator identification signal can be derived, and/or
(d1) providing the patient identification signal by an active interaction of the patient or a third person, preferably by entering the patient identification signal or a patient identifier from which the patient identification signal can be derived.

7. The method of any one of the above claims, wherein step (b) and/or (d) comprises/comprise a step of:
(b2) automatically providing the operator identification signal without requiring an active interaction of the operator or a third person, preferably by automatically reading out the operator identification signal, or an operator identifier from which the operator identification signal can be derived, from an operator identification device preferably carried by the operator, and/or
(b2) automatically providing the patient identification signal without requiring an active interaction of the patient or a third person, preferably by automatically reading out the patient identification signal, or a patient identifier from which the patient identification signal can be derived, from a patient identification device preferably carried by the patient.

8. The method of claim 7, wherein step (b2) and/or (d2) is/are executed by establishing a communication for reading out the operator identification signal and/or the patient identification signal, or an operator identifier and/or a patient identifier from which the operator identification signal and/or the patient identification signal can be derived.

9. A device for providing an identified measuring signal (14), comprising:
means adapted for receiving from a measuring device (12) a measuring signal representative for a physiological parameter of a patient,
means (11) adapted for receiving from an operator identification device (10) an operator identification signal for identifying an operator performing or helping to perform the measurement, and
means (13) for combining and/or associating the measured signal with the operator identification signal for providing an operator identified measuring signal (14).

10. The device of claim 9, comprising:
means (110) adapted for receiving from a patient identification device (100) a patient identification signal for identifying the patient, and
means (13) for combining and/or associating the measured signal with the patient identification signal for providing a combined patient operator identified measuring signal (14).

11. The device of claim 9 or 10, comprising:
means (17; 170) for probing whether the operator identification signal and/or the patient identification signal is/are valid.

12. A system for measuring a physiological parameter of a patient, comprising:
measuring means (12) for measuring a signal representative for the physiological parameter of the patient,
operator identification means (10) for providing an operator identification signal for identifying an operator performing or helping to perform the measurement, and
the device according to claim 9 or 11 for combining and/or associating the measured signal with the operator identification signal for identifying the measured signal with respect to the operator.

13. The system of claim 12, comprising:
patient identification means (100) for providing a patient identification signal for identifying the patient, and
the device according to claim 10 or 11 for combining and/or associating the measured signal with the patient identification signal for identifying the measured signal with respect to the patient.

14. The system of claim 12 or 13, wherein the operator identification means (10) and/or the patient identification means (100) comprises/comprise:
means for providing the operator identification signal and/or the patient identification signal by an active interaction of the operator or a third person and/or of the patient or a third person, preferably by entering the operator identification signal and/or the patient identification signal or an operator identifier and/or a patient identifier from which the operator identification signal and/or the patient identification signal can be derived.

15. The system of claim 12 or 13, wherein the operator identification means (10) and/or the patient identification means (100) comprises/comprise:
means for automatically providing the operator identification signal and/or the patient identification signal without requiring an active interaction of the operator or a third person and/or of the patient or a third person, preferably by automatically reading out the operator identification signal and/or the patient identification signal, or an operator identifier and/or a patient identifier from which the operator identification signal and/or the patient identification signal can be derived, from an operator identification device and/or a patient identification device preferably carried by the operator and/or the patient.

16. The system of claim 15, wherein the operator identification means (10) and/or the patient identification means (100) comprises/comprise:
means for establishing a communication with the device for reading out the operator identification signal and/or the patient identification signal, or an operator identifier and /or a patient identifier from which the operator identification signal and/or the patient identification signal can be derived.

17. The system of any one of the claims 12-16, wherein the operator identification means (10) and/or the patient identification means (100) comprises/comprise:
means adapted for receiving a user initiated entry of the operator identification signal and/or the patient identification signal or a corresponding operator identifier and/or patient identifier, preferably a key-code or a smart card, and/or
means for automatically sensing the operator identification signal and/or the patient identification signal or a corresponding operator identifier and/or patient identifier, preferably by means of a tag and/or a transponder worn or otherwise carried by the operator and/or the patient, and/or
means for evaluating the characteristic of a biological feature of the operator and/or of the patient, preferably fingerprint and/or voice.

18. The system of any one of the claims 12-17, wherein the device comprises:
an operator identification reader (16) and/or a patient identification reader (160) for establishing a communication with the operator identification means (10) and/or the patient identification means (100) for reading out the operator identification signal and/or the patient identification signal or a corresponding operator identifier and/or patient identifier therefrom.

19. The system of any one of the claims 12-18, wherein the device comprises:
a sensing device capable of reading the operator identification signal and/or the patient identification signal or a corresponding operator identifier and/or patient identifier from the operator identification means (10) and/or the patient identification means (100).

20. Use of an operator identification means (10), preferably a smart card, for providing an operator identification signal for identifying an operator, for combining and/or associating a measured signal representative for a physiological parameter of a patient with the operator identification signal for identifying the measured signal with respect to the operator.

21. Use of the characteristic of a biological feature of an operator, preferably fingerprint and/or voice, as an operator identification signal for identifying the operator, for combining and/or associating a measured signal representative for a physiological parameter of a patient with the operator identification signal for identifying the measured signal with respect to the operator.
